# EUROPEAN PATENT APPLICATION

(11) **EP 2 229 902 A2**
(43) Date of publication of application: **22.09.2010**
(21) Application number: 10460011.9
(22) Date of filing: 19.03.2010
(51) Int. Cl.: A61B 17/70

(54) **Transpedicular spinal stabilizer**

(30) Priority: 20.03.2009 PL 38756709
(71) Applicant: Uniwersytet Technologiczni-Przyrodniczy Im. Jana I Jedrzeja Sniadeckich W Bydgoszcz, 85-225 Bydgoszcz (PL)
(72) Inventor: Uniwersytet Technologiczni-Przyrodniczy Im. Jana I Jedrzeja Sniadeckich W Bydgoszcz, 85-225 Bydgoszcz (PL)
(74) Representative: Jankowski, Piotr

(57) **Abstract**

The transpedicular stabilizer according to the invention **characterized in that** stiffness related to the shift of the external sleeve 4 in relation to the pin 6 varies in the direction of planes /A/ and /B/ and is limited by the gap /L3/ and the elastic sleeve /5/, whereas between internal planes /A/ of the external sleeve /4/ and the pin /6/ a gap /L1/ is created with a size within the range of 0.05-0.2 of the width /a/ of the insert /2/, and between surfaces of the external sleeve /4/ and the pin /6/ there is a gap /L2/ > 2/L1/ and a gap /L3/ > width /a/ of the insert /2/.

## Description

The invention relates to transpedicular spinal stabilizer.

Technical literature describes a stabilizer for correction of spinal curvature by means of derotation of vertebrae and a change of scoliosis into natural kyphosis with hooks and lateral stabilizing rods and the Ronsard stabilizer in a form of elastic bonds affixed around processes without the metal anchor, and the GRAF dynamic stabilizer comprising a ligament located between transpedicular screws in two adjacent vertebrae, whereas the FASS system is similar to the GRAF stabilizer with an additional plastic beam for adjusting initial stress and consequently stiffness of the stabilizer.

The DSSII stabilization system is based on structural elements made of springs and titanium rods affixed to a vertebra with transpedicular screws; the CD stabilizer, fixed by means of various systems stabilizes the damaged spine, whereas the design of the stabilizer for correction of spinal curvature by means of derotation of vertebrae and a change of scoliosis into natural kyphosis is based on hooks and lateral stabilizing rods.

A range of plate stabilizers are known, including the ZESPOL plate stabilizer used for stabilizing the thoracic spine, whereas the Bronsard stabilizer is based on elastic bonds placed around processes without the metal anchor.

Patent no. 163120 describes the plate stabilizer of the thoracic and lumbar spine that in principle focuses on the shape of plates with elongated holes in a long axis, grooves on the plate surface and inserts in the holes, whereas application no. 365719 describes a stabilizer in a form of a screw that has a fixing head with a press element and a blind nut joined together permanently.

The transpedicular stabilizer according to the invention is **characterized in that** the stiffness related to the shift of the external part in relation to the internal part of the pin is different in the direction of planes A and B and limited by gap L3 and the elastic sleeve, whereas between internal planes A of external and internal part a gap L1 is created with a size within the range of 0.05-0.2 of the pin width a and between surfaces of the external and internal part there is gap L2 > 2L1 and gap L3 > pin width.

A technical advantage of the transpedicular stabilizer is the possibility to obtain qualitatively different mechanical characteristics in parallel planes, which results from load transfer mechanisms.

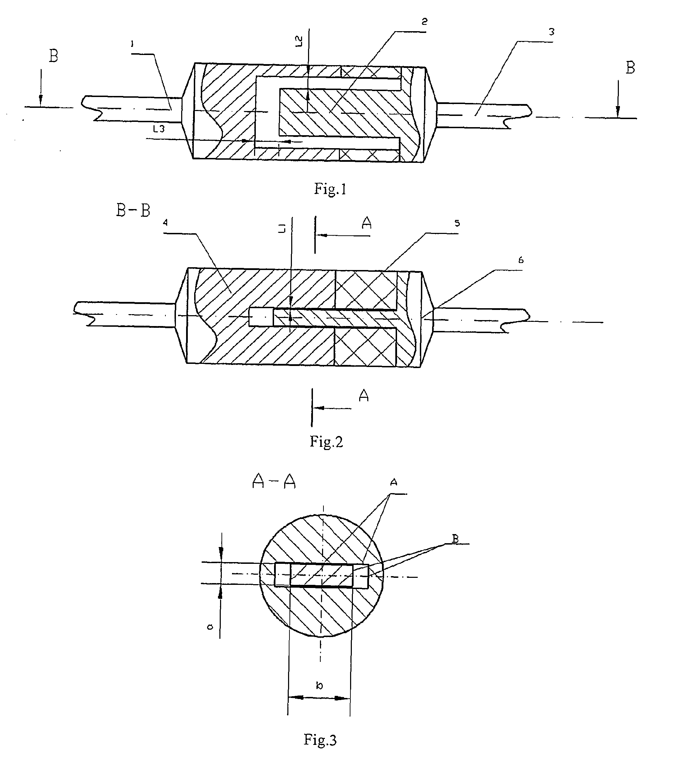

The invention is presented in a schematic way in drawings of an embodiment of the invention; fig.1 shows axial longitudinal section of the stabilizer, fig.2 shows longitudinal section, fig. 3 shows A-A section of a part of the stabilizer

The transpedicular stabilizer comprises the fixing part 1, the internal sleeve 4, the elastic sleeve 5 and the pin 6 with the insert 2, with dimensions a and b and the fixing part 3.

Between horizontal A and vertical B internal planes of the external sleeve 4 with the cuboidal hole and the insert 2 a horizontal gap L₁ is created with a size within the range of 0.05-0.2 of the insert 2 width, whereas between surfaces A of the external sleeve 4 and the insert 2 there is a gap L₂ > 2L₁ and a gap L₃ > width a, of the external sleeve 4.
The external sleeve 4 has a hole, rectangular in section and is joined permanently with the fixing part 1.

The pin 6 has the fixing part 3 on one end; this fixing part has the shape of a cylinder with a smaller diameter and is joined permanently with the pin. On the other end, the pin has the insert 2 cuboidal in shape.

The operation of the transpedicular stabilizer is based on locking the insert 2 when the spine is bent forward or backward as a result of the pressure on the internal plane of the external sleeve 4.

Small size of the gap L1 results in high stiffness of the stabilizer.
When the spine is bent left or right, the large space L1 results in low stiffness of the stabilizer.

With the maximum tilt, the space L3 is reduced until the movement of two parts of the stabilizer is blocked.

## Claims

1. The transpedicular stabilizer according to the invention is **characterized in that** stiffness related to the shift of the external sleeve /4/ in relation to the internal part of the pin /6/ varies in the direction of planes /A/ and /B/ and is limited by the gap /L3/ and the elastic sleeve /5/, whereas between internal planes /A/ of the external sleeve /4/ and the pin /6/ a gap /L1/ is created with a size within the range of 0.05-0.2 of the width /a/ of the insert /2/, and between surfaces of the external sleeve /4/ and the pin /6/ there is a gap /L2/ > 2 /L1/ and a gap /L3/ > width /a/ of the insert /2/.
